(19) Europäisches Patentamt / European Patent Office / Office européen des brevets

(11) **EP 2 459 306 B1**

(12) **FASCICULE DE BREVET EUROPEEN**

(45) Date de publication et mention de la délivrance du brevet:
**27.12.2017 Bulletin 2017/52**

(21) Numéro de dépôt: **10752890.3**

(22) Date de dépôt: **28.07.2010**

(51) Int Cl.:
**B01J 13/18** *(2006.01)*

(86) Numéro de dépôt international:
**PCT/FR2010/051604**

(87) Numéro de publication internationale:
**WO 2011/012813 (03.02.2011 Gazette 2011/05)**

(54) **MATERIAU CŒUR ECORCE, SON PROCEDE DE PREPARATION ET UTILISATION POUR LA DELIVRANCE THERMOSTIMULEE DE SUBSTANCES D'INTERET**

KERN-/HÜLLE-MATERIAL, HERSTELLUNGSVERFAHREN DAFÜR UND VERWENDUNG FÜR DIE WÄRMESTIMULIERTE HERSTELLUNG VON BESTIMMTEN STOFFEN

CORE-SHELL MATERIAL, METHOD FOR PREPARING SAME, AND USE THEREOF FOR THE THERMOSTIMULATED GENERATION OF SUBSTANCES OF INTEREST

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**

(30) Priorité: **31.07.2009 FR 0955417**

(43) Date de publication de la demande:
**06.06.2012 Bulletin 2012/23**

(73) Titulaire: **Centre National de la Recherche Scientifique**
**75016 Paris (FR)**

(72) Inventeurs:
• **SCHMITT, Véronique**
  **F-33400 Talence (FR)**
• **DESTRIBATS, Mathieu**
  **F-33000 Bordeaux (FR)**
• **BACKOV, Rénal**
  **F-33200 Bordeaux-Cauderan (FR)**

(74) Mandataire: **Goulard, Sophie et al**
**Ipsilon**
**Le Centralis**
**63, avenue du Général Leclerc**
**92340 Bourg-la-Reine (FR)**

(56) Documents cités:
**WO-A2-2008/072239     FR-A1- 2 865 416**

• **FRELICHOWSKA J ET AL: "Topical delivery of lipophilic drugs from o/w Pickering emulsions" INTERNATIONAL JOURNAL OF PHARMACEUTICS, ELSEVIER BV, NL LNKD-DOI:10.1016/J.IJPHARM.2008.12.017, vol. 371, no. 1-2, 17 avril 2009 (2009-04-17), pages 56-63, XP026071362 ISSN: 0378-5173 [extrait le 2009-03-25]**

**Description**

La présente invention est relative à un matériau constitué d'une enveloppe de silice renfermant un coeur de cire, à son procédé de préparation, à son utilisation pour la délivrance thermostimulée de substances actives, ainsi qu'aux compositions renfermant un tel matériau.

Il peut être utile d'encapsuler des molécules d'intérêt telles que des médicaments, des colorants, des pigments, des réactifs, des parfums, des pesticides, etc..., pour les protéger des agressions extérieures, notamment de l'oxydation, pour les acheminer vers un lieu d'administration où elles pourront être délivrées ou bien encore pour les stocker avant une utilisation dans des conditions où elles seront libérées de leur capsule sous l'influence d'un stimulus externe. Une des premières applications de la micro encapsulation été la mise au point d'un papier copiant sans carbone commercialisé à la fin des années 60 dans lequel des microcapsules emprisonnant une encre étaient présentes sur le verso d'une feuille de papier de façon à libérer l'encre par rupture des capsules sous la pression exercée par la pointe d'un stylo lors de l'écriture. De nos jours, l'encapsulation se développe dans différents secteurs industriels tels que les industries pharmaceutique, cosmétique, alimentaire, textile et agricole. Les capsules et microcapsules deviennent de plus en plus sophistiquées, notamment dans le domaine pharmaceutique où elles permettent de faire de la délivrance contrôlée et/ou ciblée de principes actifs

Différents types et morphologies de capsules et microcapsules ont déjà été proposées tels que par exemple des capsules protéiques, des cyclodextrines, des liposomes, des vésicules lamellaires concentrées, des émulsions doubles, des colloidosomes, des microcapsules à enveloppes de silice, des nanocapsules de silice et de polymères thermosensibles tel que le poly(*N*-isopropylacrylamide (PNIPAM), des microsphères d'hydrogel thermosensible, des microsphères de PNIPAM-Polylactide, etc....De nombreuses méthodes permettant de préparer ces différents type de capsules et microcapsules ont également été développées et mises au point durant ces dernières années, telles que par exemple et de façon non exhaustive la précipitation de polymères par séparation de phase, le dépôt d'électrolyte couche par couche, la polymérisation par polycondensation interfaciale, etc... WO 2008/072239 A2 décrit des nanocapsules solides substantiellement sphériques constituées d'une enveloppe continue comprenant un oxyde de silicium, ladite enveloppe forme une capsule encapsulant une huile de paraffine et du Rouge Nil. Les particules ont un d90 de 190 nm et l'enveloppe des particules a une épaisseur de 4-8 nm. L'inconvénient des techniques déjà connues est que la libération des molécules d'intérêt à partir des capsules et microcapsules proposées dans l'art antérieur est le plus souvent lente et progressive, c'est-à-dire prolongée dans le temps.

Il n'existe pas à ce jour de système autorisant une libération rapide et totale de molécules d'intérêt sous l'effet d'un stimulus extérieur en conditions douces.

Le but de la présente invention est donc de proposer une capsule permettant d'encapsuler une ou plusieurs molécules d'intérêt qui puisse(nt) être libérée(s) de façon rapide et totale sous l'influence d'un stimulus extérieur, et en particulier d'une augmentation de la température.

La présente invention a pour objet un matériau sous forme de particules solides constituées d'une enveloppe continue comprenant au moins un oxyde de silicium, ladite enveloppe emprisonnant au moins une phase grasse, ledit matériau étant caractérisé en ce que ladite phase grasse est solide à la température de stockage dudit matériau et comprend majoritairement une huile cristallisable ayant une température de fusion ($T_F$) inférieure à 100°C et au moins une substance d'intérêt, et en ce que lesdites particules ont un diamètre variant de 1 $\mu$m à 1 cm.

Selon la présente invention, on entend par « température de stockage dudit matériau », la température à laquelle le matériau conforme à la présente invention est conservé avant son utilisation. Cette température est toujours inférieure au point de fusion de l'huile cristallisable contenue dans la phase grasse.

Le matériau conforme à la présente invention présente la particularité suivante : lorsque l'on soumet le matériau à une température supérieure à la température de fusion de l'huile cristallisable, on observe une expansion thermique de la phase grasse entraînant la rupture de l'enveloppe de silice et la libération rapide et totale de la phase grasse en fusion (c'est-à-dire à l'état liquide) comprenant la ou les substances d'intérêt. Ce résultat est tout à fait surprenant dans la mesure ou l'oxyde de silicium entrant dans la constitution de l'enveloppe est connu pour être un isolant thermique.

On entend dans le cadre de cet exposé par le terme « huile cristallisable », les matières grasses et les mélanges de matières grasses, d'origine naturelle (animale ou végétale) ou synthétique, dont le point de fusion est supérieur à 15°C, de préférence dont le point de fusion varie de 20 à 100°C environ, et en particulier de 20 à 50°C environ. Tous les points de fusion mentionnés dans la description de la présente demande font référence à des points de fusion déterminés par calorimétrie différentielle à balayage à pression atmosphérique ou « *Differential Scanning Calorimetry* » (DSC) en anglais.

L'huile cristallisable forme une partie majoritaire de la phase grasse et peut même, outre la ou les substances d'intérêt, être l'unique constituant de celle-ci. Généralement, l'huile cristallisable représente 50 à 99,9 % en poids environ, de préférence de 75 à 99,9 % en poids environ de la phase grasse.

Le choix de l'huile cristallisable dépend naturellement de l'application envisagée pour le matériau et donc de la température à laquelle on souhaite observer l'expansion thermique de la phase grasse et par voie de conséquence la rupture de l'enveloppe de silice. Parmi les huiles cristallisables utilisables selon l'invention, on peut notamment citer les paraffines telles que les paraffines ayant un point de fusion entre 42 et 44°C ou entre 46 et 48°C [RN- 8002-74-2] vendues par la société Merck, ; les triglycérides ; les acides gras ; les colophanes ; les cires (alcanes longs) tels que l'eicosane et l'octadécane ; les huiles végétales hydrogénées ainsi que leurs mélanges ; et les bitumes synthétiques. Ces huiles peuvent être utilisées seules ou en mélanges.

Le matériau conforme à la présente invention se présente de préférence sous la forme d'une poudre de particules sphériques ou sensiblement sphériques.

Le diamètre des particules varie de préférence de 5 $\mu$m à 500 $\mu$m environ, et encore plus préférentiellement de 10 à 200 $\mu$m.

L'enveloppe de silice doit avoir une épaisseur suffisante pour avoir une résistance mécanique permettant l'encapsulation de la phase grasse, tout en étant assez fine pour pouvoir se rompre lors d'une élévation de la température à une température supérieure à la température de fusion de la phase grasse constituant le coeur du matériau. L'épaisseur de l'enveloppe de silice varie généralement de 0,1 à 2 $\mu$m environ, et préférentiellement de 0,2 à 2 $\mu$m environ.

En plus de l'oxyde de silicium, l'enveloppe peut en outre comprendre un ou plusieurs oxydes métalliques de formule $MeO_2$ dans laquelle Me est un métal choisi parmi Zr, Ti, Th, Nb, Ta, V, W et Al. Dans ce cas, l'enveloppe est une matrice mixte de type $SiO_2$-$MeO_2$ dans laquelle la teneur en $MeO_2$ reste minoritaire par rapport à la teneur en oxyde de silicium, préférentiellement la teneur en $MeO_2$ représente de 1% à 40% massique, plus particulièrement de 5% à 30% massique par rapport au poids total de l'enveloppe.

La phase grasse du matériau conforme à l'invention peut renfermer tout type de substances d'intérêt, que celles-ci soient lipophiles ou hydrophiles. Ainsi lorsque la ou les substances d'intérêt sont lipophiles, la phase grasse les contient sous forme solubilisée et lorsque la ou les substances d'intérêt sont hydrophiles, la phase grasse les contient sous forme dispersée (directement dans l'huile cristallisable ou dans une fraction d'eau dispersée au sein de la phase grasse (émulsion double)). Il peut également s'agir de particules solides.

Parmi les substances d'intérêt pouvant être incorporées dans la phase grasse du matériau conforme à la présente invention, on peut notamment citer les médicaments (principes actifs), les principes actifs utilisables en cosmétique, les réactifs chimiques, les colorants, les pigments, les encres, etc ...

A titre d'exemples de médicaments, on peut mentionner les bactéricides tels que les antiseptiques et les antibiotiques, les anti inflammatoires, les analgésiques, les laxatifs locaux, les hormones, les protéines, etc...

A titre d'exemples de principes actifs cosmétiques, on peut notamment citer les vitamines, les filtres solaires, les antioxydants tels que les antiradicalaires comme la superoxyde dismutase, les parfums, les agents absorbeurs d'odeur, les agents déodorants, les agents anti transpirants, les colorants, les pigments, les émollients, les agents hydratants, etc...

A titre d'exemples de réactifs chimiques, on peut notamment citer, les réactifs colorés, les indicateurs colorés tels que les indicateurs de pH, les catalyseurs, les amorceurs de polymérisation, les monomères, les complexants, etc...

La ou les substances d'intérêt peut représenter soit la totalité soit une partie du complément à 100 % de la phase grasse. La ou les substances d'intérêt représentent généralement de 0,1 à 50 % en poids environ, et préférentiellement de 0,1 à 25% en poids environ du poids total de la phase grasse.

La phase grasse peut en outre renfermer un ou plusieurs additifs classiquement utilisés dans les émulsions et parmi lesquels on peut notamment mentionner à titre d'exemple les protecteurs ou de conservation de la substance d'intérêt, tels que les anti-oxydants, les agents anti-UV.

L'invention a également pour objet un procédé de préparation du matériau tel que défini ci-dessus. Ce procédé est caractérisé en ce qu'il comporte les étapes suivantes consistant :

1) dans une première étape, à porter une phase grasse comprenant majoritairement une huile cristallisable (HC) solide ayant une température de fusion $T_F$ inférieure à 100°C à une température $T_{HC}$ telle que $T_{HC}$ est supérieure à $T_F$, pour obtenir une huile cristallisable à l'état liquide ;

2) dans une deuxième étape, à incorporer à la phase grasse à l'état liquide au moins une substance d'intérêt ;

3) dans une troisième étape, à mettre en contact ladite phase grasse à l'état liquide avec une phase aqueuse (PA) préalablement portée à une température $T_{PA}$ telle que $T_{PA}$ est supérieure à $T_F$, ladite phase aqueuse renfermant en outre des particules solides colloïdales ;

4) dans une quatrième étape, à soumettre le mélange liquide résultant de la troisième étape à une agitation mécanique pour obtenir une émulsion huile-dans eau formée de gouttelettes de phase grasse à l'état liquide dispersées dans la phase aqueuse continue et dans laquelle les particules solides colloïdales sont présentes à l'interface formée entre la phase aqueuse continue et les gouttelettes de phase grasse dispersées ;

5) dans une cinquième étape, à porter ladite émulsion H/E à une température $T_{H/E}$ telle que $T_{H/E}$ est inférieure à $T_F$ pour provoquer la solidification de la phase grasse et obtenir une émulsion H/E formée de globules de phase grasse à l'état solide, lesdits globules étant dispersés dans la phase aqueuse ;

6) dans une sixième étape, à former une enveloppe continue comprenant au moins un oxyde de silicium autour desdits globules par ajout, dans la phase aqueuse de l'émulsion, et sous agitation mécanique, d'au moins un précurseur d'oxyde de silicium et d'une quantité suffisante d'au moins un acide pour amener la phase aqueuse à pH inférieur ou égal à 4 pour obtenir ledit matériau ;

7) dans une septième étape, à séparer ledit matériau de la phase aqueuse.

Les particules solides colloïdales présentes dans la phase aqueuse lors de la troisième étape peuvent être minérales ou organiques. De préférence, il s'agit de particules minérales. Les particules solides colloïdales sont de préférence des particules minérales choisies dans le groupe des oxydes, hydroxydes et sulfates de métaux. Parmi de tels oxydes, on peut tout particulièrement citer les oxydes de silicium, de titane, de zirconium et, de fer, ainsi que leurs sels tels que les silicates (par exemple les argiles). Enfin, on peut citer les particules colloïdales de carbone. Parmi les particules solides colloïdales organiques, on peut citer notamment les particules polymériques, par exemple des particules de latex.

Afin d'être colloïdales, les particules solides présentent généralement une taille inférieure à quelques micromètres. Ainsi les particules présentent généralement une taille moyenne comprise entre 5 et 5000 nm, et de préférence entre 5 et 500 nm.

Selon une forme de réalisation particulièrement préférée de l'invention, les particules solides colloïdales sont choisies parmi les nanoparticules d'oxyde de silicium. A titre d'exemple, on peut notamment citer les produits vendus sous la dénomination commerciale Aerosil ® par la société Evonik Degussa.

La quantité de particules solides colloïdales varie généralement de 0,01 % à 10%, en particulier de 0,1% à 5% en poids par rapport au poids total de la phase grasse. Avantageusement, la quantité de particules solides colloïdales présentes dans la phase aqueuse continue varie en fonction de la taille moyenne en volume des gouttelettes de la phase grasse souhaitées dans l'émulsion et dont le diamètre moyen varie de 1 $\mu$m à 1 cm, de préférence entre 5 et 500 $\mu$m, et encore plus préférentiellement entre 10 et 200 $\mu$m environ.

Par ailleurs, les particules solides colloïdales présentent généralement une surface hydrophile et chargée, ce qui ne favorise pas leur adsorption à la surface des gouttelettes de la phase grasse dispersée.

Ainsi, et selon une forme de réalisation préférée de l'invention, les particules solides colloïdales sont fonctionnalisées en surface pour favoriser leur adsorption à l'interface formée entre la phase aqueuse continue et les gouttelettes de phase grasse dispersées.

Les particules solides colloïdales peuvent ainsi être fonctionnalisées par des composés liés à leur surface par des liaisons covalentes. Cela peut être réalisé par traitement préalable des particules, en particulier par greffage chimique d'un composé comportant des groupements hydrophobes tel qu'un trialkoxysilane de formule $R-Si-(OR')_3$, dans laquelle R est un alkyle linéaire ou ramifié en $C_1$ à $C_{12}$, en particulier de $C_2$ à $C_{10}$, tout particulièrement n-octyle, portant éventuellement un groupe amino et R', identique ou différent de R, est un groupe alkyle linéaire ou ramifié en $C_1$ à $C_{12}$, en particulier de $C_1$ à $C_6$, et tout particulièrement éthyle.

Les particules solides colloïdales peuvent également être fonctionnalisées par adsorption de molécules de tensioactif à leur surface qui permettent de leur conférer une certaine hydrophobie, l'extrémité hydrophile du tensioactif étant adsorbée sur la surface des particules. Les tensioactifs utilisables pour fonctionnaliser les particules sont de préférence des tensioactifs cationiques ou anioniques.

Parmi ces tensioactifs, on préfère en particulier les alkylsulfates de sodium tels qu'en particulier le dodécylsulfate de sodium (SDS) et les bromures d'alkyltriméthylammonium.

Le tensioactif est de préférence choisi parmi les tensioactifs de charge opposée à celle de la surface des particules solides colloïdales. Ce choix permet de favoriser l'adsorption du tensioactif à la surface des particules.

A titre d'exemple de particules fonctionnalisées par un tensioactif, on peut notamment citer les nanoparticules de silice dont la surface est fonctionnalisée par un ammonium quaternaire telles que celles vendues sous la dénomination Aerosil® A380 par la société Evonik Degussa, de diamètre 7 nm, et dont la surface est fonctionnalisée par le bromure de cétyl-triméthylammonium (CTAB).

La fonctionnalisation des particules solides colloïdales par un tensioactif peut également être réalisée *in situ,* c'est-à-dire lors de leur introduction dans la phase aqueuse continue de l'émulsion. Dans ce cas, la phase aqueuse continue de l'émulsion renferme en outre ledit tensioactif en une quantité préférentiellement inférieure à la concentration micellaire critique (CMC), celui-ci venant alors s'adsorber à la surface des particules lorsque celles-ci sont dans la phase aqueuse de l'émulsion. De préférence, la quantité de tensioactif varie de 1/200$^{ème}$ à 1/3 de la CMC.

La phase aqueuse continue comprend principalement de l'eau et éventuellement un alcool, tel que le méthanol, l'éthanol, l'isopropanol, ou le butanol, de préférence l'éthanol.

L'agitation mécanique réalisée lors de la quatrième étape peut notamment être réalisée dans un dispositif destiné à émulsionner tel que par exemple dans des dispositifs vendus sous les dénominations commerciales Ultra-Turrax® ou Rayneri®.

La distribution de taille des gouttelettes de la phase grasse dans l'émulsion H/E est généralement étroite (U<40%).

Lors de la sixième étape, l'ajout d'au moins un précurseur d'oxyde de silicium à pH acide provoque la condensation dudit précurseur à l'interface des globules de phase grasse à l'état solide et la formation de l'enveloppe.

Les précurseurs d'oxyde de silicium peuvent être choisis parmi les alkoxydes de silicium et en particulier parmi le tetraméthoxyorthosilane (TMOS), le tetraéthoxyorthosilane (TEOS), le diméthyldiéthoxysilane (DMDES), le (3-mercaptopropyl)triméthoxysilane, le (3-aminopropyl)triéthoxysilane, le N-(3-triméthoxysilylpropyl)pyrrole, le 3-(2,4-dinitrophénylamino)-propyltriéthoxysilane, le N-(2-aminoéthyl)-3-aminopropyltriméthoxysilane, le phényltriéthoxysilane, le méthyltriéthoxysilane et leurs mélanges. Parmi ces précurseurs, le TEOS est particulièrement préféré. Ces précurseurs peuvent être substitués, de manière totale ou partielle, par des sols de silicate.

L'épaisseur de l'enveloppe dépend de la quantité de précurseurs d'oxyde de silicium utilisée lors de la sixième étape et du diamètre des globules de la phase grasse dispersée. Cette quantité est exprimée par rapport à la surface totale en $m^2$ des globules de la phase dispersée de l'émulsion.

Selon une forme de réalisation préférée de l'invention, la quantité de précurseur d'oxyde de silicium varie de 0,05 à 4 $M/m^2$, et encore plus préférentiellement de 0,2 à 2,2 M par $m^2$ de surface des globules de la phase dispersée de l'émulsion.

Pour atteindre les plus grandes épaisseurs de l'enveloppe, la sixième étape peut être réalisée plusieurs fois jusqu'à obtenir l'épaisseur désirée.

Lorsque l'enveloppe du matériau conforme à l'invention comprend, outre l'oxyde de silicium, un oxyde métallique, on ajoute alors également à la phase aqueuse de l'émulsion au moins un précurseur d'un oxyde métallique de formule $MeO_2$, ledit précurseur étant choisi parmi les alcoxydes, les chlorures ou les nitrates des métaux choisis parmi Zr, Ti, Th, Nb, Ta, V, W et Al.

Lorsqu'ils sont utilisés, la quantité de ces précurseurs d'oxyde métallique de formule $MeO_2$ varie de 0,001 M à 1 M, et préférentiellement de 0,01 à 0,6 M par $m^2$ de surface des globules de la phase dispersée de l'émulsion.

Le pH de la phase aqueuse lors de la sixième étape varie de préférence de 0,01 à 4, et encore plus préférentiellement de 0,1 à 2,1.

L'acide utilisé pour ajuster le pH de la phase aqueuse peut être choisi parmi les acides minéraux et organiques parmi lesquels on peut en particulier citer l'acide chlorhydrique, l'acide acétique, l'acide nitrique et l'acide sulfurique.

Lors de la septième étape, le matériau conforme à l'invention peut être séparé de la phase aqueuse et récupéré par toute technique classique de séparation connue de l'homme du métier, telle que la filtration, la centrifugation et l'utilisation de tamis. Il est ensuite de préférence lavé, par exemple à l'eau, puis séché par exemple par lyophilisation pour donner une poudre.

Le matériau ainsi obtenu est stable au stockage pendant plusieurs mois à condition que la température de stockage soit inférieure à la température $T_F$ de la phase grasse emprisonnée dans l'enveloppe.

Le matériau conforme à l'invention peut être utilisé sous forme de poudre ou de dispersion dans un solvant pour délivrer la ou les substances d'intérêt présente(s) dans la phase grasse solide emprisonnée dans l'enveloppe à base d'oxyde de silicium.

L'invention a donc également pour objet l'utilisation d'un matériau conforme à l'invention et tel que décrit précédemment pour la délivrance thermostimulée d'au moins une substance d'intérêt.

La délivrance de la substance d'intérêt est obtenue par rupture de l'enveloppe sous l'effet d'une élévation de la température à une température de délivrance $T_D$ telle que $T_D > T_F$.

A titre d'exemple, et lorsque la substance d'intérêt est un médicament, l'huile cristallisable présente dans la phase grasse est de préférence choisie parmi les huiles cristallisables ayant un point de fusion inférieur à 37C°. Ainsi, lorsque ledit matériau est incorporé dans une composition pharmaceutique et que cette composition est administrée à un patient, par exemple par voie orale, la composition ingérée va se trouver à la température du corps, en général 37°C ou plus, ce qui va entraîner la fusion de la phase grasse et son expansion volumique et ainsi la rupture de l'enveloppe de silice et la délivrance du médicament.

Selon un autre exemple, la substance d'intérêt est un principe actif cosmétique et le matériau fait partie des composants d'une composition cosmétique à application topique, telle qu'une poudre, une crème ou un gel. L'échauffement de la phase grasse du matériau à une température supérieure à $T_F$ peut dans ce cas être provoqué par un frottement local lors de l'étalement de la composition cosmétique, ce qui induit un échauffement local entraînant la rupture de l'enveloppe et la libération locale de la substance d'intérêt. Si la composition cosmétique se présente sous la forme d'une poudre, son application par étalement peut s'accompagner d'un changement de texture (transformation de la poudre en une composition ayant un toucher gras dû à la rupture de l'enveloppe).

A titre d'autres exemples d'utilisation du matériau conforme à l'invention, on peut notamment citer :

- l'utilisation comme témoin de conservation d'un objet, par exemple d'un aliment contenu dans un emballage alimentaire, à une température inférieure à une température maximale de conservation. Dans ce cas, l'emballage alimentaire comprend un témoin de conservation renfermant un matériau conforme à l'invention dans lequel la phase grasse contient un réactif dont la couleur change ou est révélée lors de la rupture de l'enveloppe par exposition de l'emballage à une température supérieure à la température maximale de conservation de l'aliment, l'huile cristallisable de la phase grasse étant choisie parmi les huiles dont le point de fusion est juste supérieur à la température maximale de conservation ;

- l'utilisation pour la délivrance d'un parfum dans l'air (action désodorisante), dans un textile, sur la peau, etc..., dès que la température de l'air, du textile ou de la peau atteint une valeur supérieure à la température de fusion de la phase grasse ;

- l'utilisation pour la délivrance d'un amorceur d'une réaction de polymérisation induite par élévation de la température ;

- l'utilisation pour la délivrance thermostimulée d'un réactif lors d'une réaction chimique ;

- l'utilisation pour la délivrance d'un agent bactéricide dans une installation industrielle, par exemple dans un conduit d'aération ou de climatisation, lorsque la température de l'installation atteint la température de prolifération des bactéries ;

- l'utilisation pour la conservation de molécules d'intérêt, etc...

L'invention a également pour objet l'utilisation du matériau tel que décrit ci-dessus, à titre d'ingrédient, pour la préparation de produits pharmaceutiques, cosmétiques ou alimentaires, ainsi que les produits pharmaceutiques, cosmétiques ou alimentaires, renfermant, à titre d'ingrédient, au moins un matériau conforme à l'invention.

Ces compositions peuvent renfermer les supports pharmaceutiques, cosmétiques ou alimentaires classiques et bien connus de l'homme du métier, ainsi qu'un ou plusieurs tensioactifs destinés à favoriser la libération de la phase grasse liquide lors de la rupture de la capsule.

La présente invention est illustrée par les exemples de réalisation suivants, auxquels elle n'est cependant pas limitée.

## EXEMPLES

Les matières premières utilisées dans les exemples qui suivent sont listées ci-après :

- paraffines en bloc ayant une plage de fusion de 42 à 44°C ou de 46 à 48°C (CAS n°8002-74-2), toutes deux vendues par la société Merck ;
- eicosane pur à 99% (point de fusion = 37C°) vendu par la société Aldrich ;
- Suppocire® DM, excipient de suppositoire, composée d'un mélange de triglycérides dont les températures de fusion s'étalent de 27 à 48°C, vendue par la société Gattefossé.
- Tetraéthoxyorthosilane (TEOS), bromure de cétyl-triméthylammonium (CTAB), société Fluka ;
- Nanoparticules de silice de 7 nm de diamètre, vendues sous la dénomination Aerosil® A380 par la société Evonik Degussa ;
- Dodécylsulfate de sodium (SDS), société Aldrich ;
- Tensioactif non ionique consistant en un mélange de polyoxyéthylènes en $C_{12}$ et $C_{10}$ à 5 moles d'oxyde d'éthylène, fourni sous la dénomination Ifralan® D205 par la société IfraChem.

Ces matières premières ont été utilisées telles que reçues des fabricants, sans purification supplémentaire.

Les matériaux obtenus ont été caractérisés à l'aide d'un microscope optique inversé vendu sous la dénomination commerciale Axiovert® X100 par la société Zeiss et équipé d'une platine chauffante de la société Mettler permettant de contrôler la température ainsi que les vitesses de chauffage et de refroidissement.

La distribution de taille des émulsions a été étudiée à l'aide d'un granulomètre vendu sous la dénomination commerciale Mastersizer Hydro MS2000 par la société Malvern Instrument. Les mesures granulométriques ont été faites à 25°C dans l'eau pure. L'intensité de la diffusion en fonction de l'angle qui a été collectée a été transformée en utilisant la théorie de Mie-Lorenz. La distribution de la taille des particules a été exprimée par leur diamètre moyen pondéré (D) et leur polydispersité (P) en appliquant les équations (1) et (2) suivantes :

$$D = \frac{\sum_i N_i D_i^3}{\sum_i N_i D_i^2} \qquad (1)$$

et

$$P = \frac{1}{\overline{D}} \frac{\sum_i N_i D_i^3 \left| \overline{D} - D_i \right|}{\sum_i N_i D_i^3} \qquad (2)$$

dans lesquelles :

- $D_i$ est le diamètre des particules,
- $N_i$ est le nombre total de gouttelettes de diamètre $D_i$,
- $\overline{D}$ est le diamètre médian, c'est-à-dire l'ouverture théorique du tamis telle que 50 % des particules, en masse, ont un diamètre supérieur et 50 % un diamètre inférieur.

Ces formules sont appliquées dans les granulomètres de la société Malvern Instrument.

Aux températures auxquelles ont été faites les mesures, les gouttelettes étaient solides et de forme sensiblement sphérique.

Les matériaux ont été observés à l'aide d'un microscope électronique à balayage (MEB) vendu sous la référence TM-1000 par la société Hitachi. Afin d'obtenir une meilleure résolution et d'estimer l'épaisseur de l'enveloppe de silice, les échantillons ont également été observés par MEB à l'aide des microscopes à balayage vendus sous les références Jeol JSM-840A et Jeol 6700F. Pour ce faire, les particules ont préalablement soit été séchées à température ambiante, soit lyophilisées à l'aide d'un appareil à lyophiliser vendu sous la dénomination Alpha 2-4 LD Plus par la société Christ. Toutes les particules ont été recouvertes d'or avant d'être observées en MEB.

**EXEMPLES**

**Exemple** 1 : **Préparation, caractérisations et étude de matériaux conformes à l'invention**

Dans cet exemple on illustre la préparation, la caractérisation et l'étude de matériaux conformes à l'invention, constitués d'une enveloppe de silice renfermant une huile cristallisable.

1) Préparation des matériaux

i) Première étape : Fonctionnalisation des particules de silice

1,38 g de nanoparticules de silice Aerosil ® A380 ont été dispersés dans 300 ml d'eau distillée, à l'aide d'une cuve à ultra sons. On a ensuite ajouté à cette dispersion, 24 mg de CTAB, cette quantité représentant environ 1/5<sup>ème</sup> de la concentration micellaire critique (CMC = $0,9.10^{-3}$ mol/l). La surface des nanoparticules de silice étant chargée négativement, le CTAB (tensioactif cationique) vient s'adsorber à la surface des particules de silice et permet ainsi de leur conférer un caractère amphiphile. On a obtenu une phase aqueuse renfermant une dispersion de nanoparticules de silice fonctionnalisées en surface.

ii) Deuxième étape : Préparation des émulsions

Selon les émulsions, dont les compositions sont précisées dans le tableau 1 ci-après, une quantité donnée de la dispersion de nanoparticules de silice fonctionnalisées obtenue ci-dessus à l'étape précédente a été diluée avec de l'eau. Cette phase aqueuse a été chauffée à une température de 65°C puis des quantités variables d'huile cristallisable telles que répertoriées dans le tableau 1 (paraffines 42-44 ; paraffine 46-48 ou eicosane), préalablement amenée à l'état liquide par chauffage, ont été introduites dans la dispersion de nanoparticules de silice sous agitation vigoureuse à l'aide d'un agitateur vendu sous la dénomination Ultra-Turrax ® T25 par la société Janke & Kunkel, équipé d'un outil de dispersion S25, en terminant par une agitation à 9000 tours/min. pendant 1 minute. On a ensuite laissé les émulsions revenir à température ambiante, sans agitation. Après refroidissement des émulsions à une température inférieure au point de fusion de l'huile cristallisable, on a ajouté du CTAB en une quantité suffisante pour atteindre la concentration micellaire critique afin d'empêcher l'agrégation des particules de cire et permettre le stockage des émulsions.

**Tableau 1**

| Emulsion | Nature Huile | $Q_H$: Quantité d'huile (en g) | $Q_{PH}$ : Quantité de phase aqueuse (en g) | $Q_{PS}$ : Quantité de particules de silice (en mg) | Rapport massique $Q_{PS}/Q_H$ (g/g) | Quantité de CTAB (en mg) | Quantité de TEOS (en M/m$^2$) |
|---|---|---|---|---|---|---|---|
| $E_{0,17}$ | Eicosane | 12,36 | 78,00 | 21 | 0,0017 | 0,37 | 1 |
| $E_{0,67}$ | Eicosane | 12,36 | 78,00 | 83 | 0,0067 | 1,44 | 1 |
| $E_{1,45}$ | Eicosane | 12,36 | 78,00 | 180 | 0,0145 | 3,12 | 1 |
| $P_{42-44}$ | Paraffine 42-44 | 12,36 | 77,00 | 180 | 0,0133 | 3,12 | 1 |
| $P_{46-48}$ | Paraffine 46-48 | 12,36 | 78,00 | 180 | 0,0145 | 3,12 | 1,7 |

iii) Troisième étape : formation de l'enveloppe de silice

Dans cette étape on a réalisé la formation de l'enveloppe de silice autour des particules de cire.

Les émulsions de particules de cire ont été diluées à 7% massique et le pH des émulsions a été ajusté à 0,2 environ, c'est-à-dire à une valeur inférieure au point isoélectrique de la silice, par ajout à la fois d'une solution d'acide chlorhydrique à 37 % en volume et d'une solution contenant 1 % massique de CTAB. Ces émulsions ont été réparties dans des tubes à essais de 10 ml.

Le TEOS a alors été ajouté au goutte à goutte aux émulsions pour atteindre la quantité notée dans le tableau 1, c'est-à-dire 1 M de TEOS par m$^2$ de surface de particules de cire stabilisées pour les émulsions $E_{0,17}$, $E_{0,67}$, $E_{1,45}$ et $P_{42-44}$ et 1,7 M /m$^2$ de surface de particules de cire stabilisées pour l'émulsion $P_{46-48}$.

On a ensuite laissé l'enveloppe de silice se former (minéralisation) sous agitation continue sur roue à 25 tours/min dans une chambre thermostatée à 25°C.

A la fin de la minéralisation, les particules de silice ont été récupérées par centrifugation et lavées plusieurs fois à l'eau distillée. Le matériau obtenu a été conservé dans l'eau pure pendant plusieurs mois. Aucune altération des capsules n'a été observée pendant cette période.

2) Résultats des caractérisations

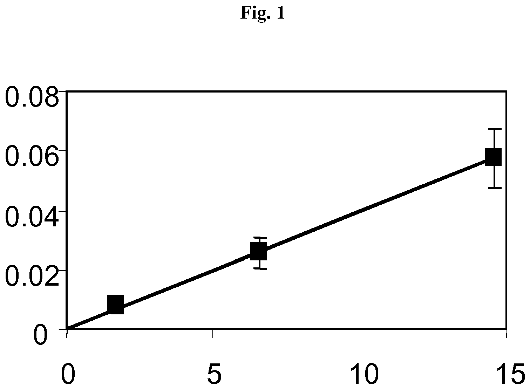

La figure 1 annexée représente l'inverse du diamètre moyen des particules du matériau conforme à l'invention en fonction du rapport entre la quantité de particules de silice fonctionnalisées et la masse d'eicosane utilisé lors de la préparation des émulsions (étape ii). Sur cette figure, l'inverse du diamètre moyen (D) des particules en $\mu$m$^{-1}$ (1/D) est fonction du rapport pondéral entre la masse de particules de silice fonctionnalisées (en mg) sur la masse d'eicosane (en g).

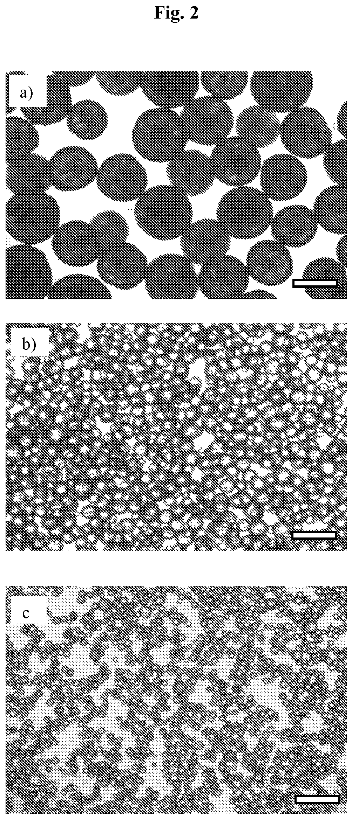

La figure 2 annexée présente des images de microscopie optique des émulsions d'eicosane dans l'eau obtenues avec différents rapports massique particules de silices fonctionnalisées/eicosane ; figure 2a : $E_{0,17}$ ; figure 2b : $E_{0,67}$ et figure 2c : $E_{1,45}$ % massique. Sur les figures 2a, 2b et 2c, la barre d'échelle correspond à 100 $\mu$m.

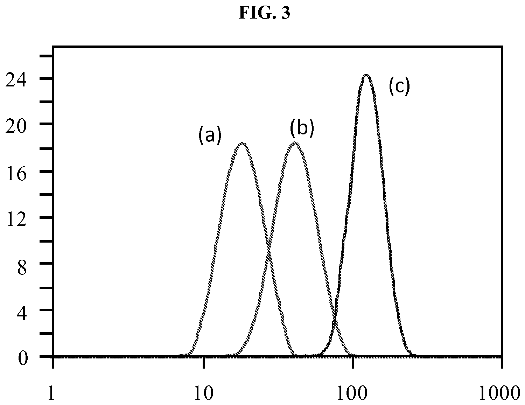

La figure 3 annexée représente la distribution granulométrique des particules des trois émulsions présentées sur les images de la figure 2 : (a) = $E_{0,17}$, (b) = $E_{0,67}$ et (c) = $E_{1,45}$ et pour lesquelles les diamètres moyens respectifs sont de 17,4 $\mu$m (a), 39 $\mu$m (b) et 121 $\mu$m (c) et les indices de polydispersité respectifs de 0,26 (a), 0,26 (b) et 0,19 (c). Sur cette figure, le volume cumulé des particules de l'émulsion, de diamètres comparables est reporté (en %) en fonction du diamètre des particules (en $\mu$m) (distribution granulométrique en volume des particules). La figure 3 représente les courbes de distribution granulométrique des trois émulsions (a), (b) et (c).

Les résultats présentés sur ces figures montrent que le procédé de préparation conforme à la présente invention conduit à des émulsions dans lesquelles la distribution granulométrique des particules est étroite (faible polydispersité). La figure 3 montre que l'on a obtenu des particules dont le diamètre moyen variait entre une dizaine et quelques centaines de $\mu$m.

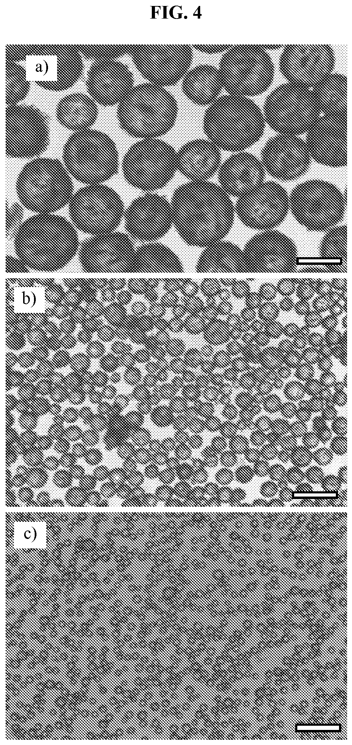

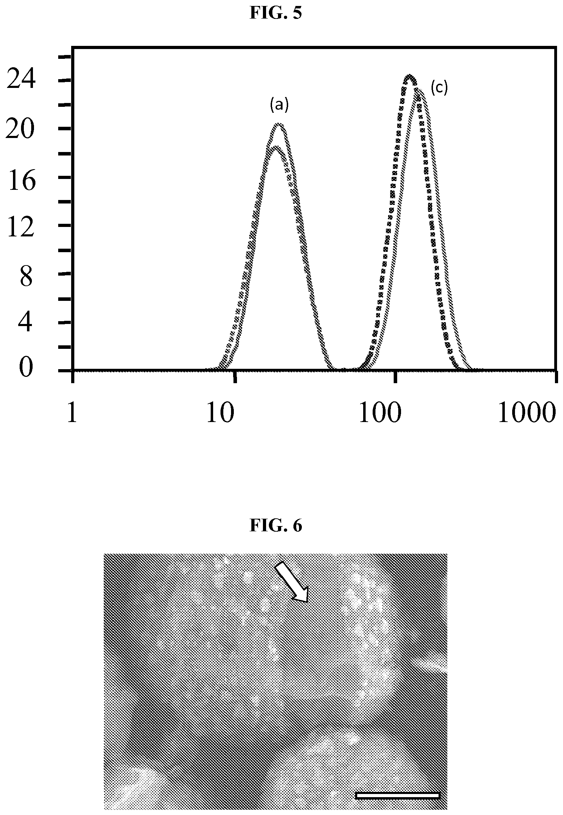

La figure 4 annexée représente des images de microscopie optique du matériau obtenu par minéralisation des émulsions (a), (b) et (c) d'eicosane stabilisées par des particules de silice fonctionnalisées de la figure 3. Sur ces figures la barre d'échelle représente 100 $\mu$m. La figure 5 annexée représente les courbe de distribution granulométrique des émulsions (a) et (c) de la figure 3, avant (courbes en trait discontinu) et après la minéralisation (courbes en trait continu).

Sur cette figure, le volume cumulé des particules de l'émulsion, de diamètres comparables est reporté (en %) en fonction du diamètre des particules (en $\mu$m). L'ensemble des résultats présentés sur les figures 4 et 5 montre que l'étape de minéralisation n'élargit pas la distribution granulométrique des particules et qu'elle n'entraîne pas de phénomène d'agglomération des particules.

La figure 6 est une photographie MEB prise lors de l'observation d'un matériau conforme à l'invention obtenu par minéralisation de l'émulsion $P_{46-48}$. Cette photographie a été obtenue après rupture de l'enveloppe par élévation de température due à la focalisation du faisceau d'électrons sur les capsules. Sur cette figure la barre d'échelle représente 10 $\mu$m et la flèche blanche pointe sur la zone de fracture provoquée par l'expansion de la cire.

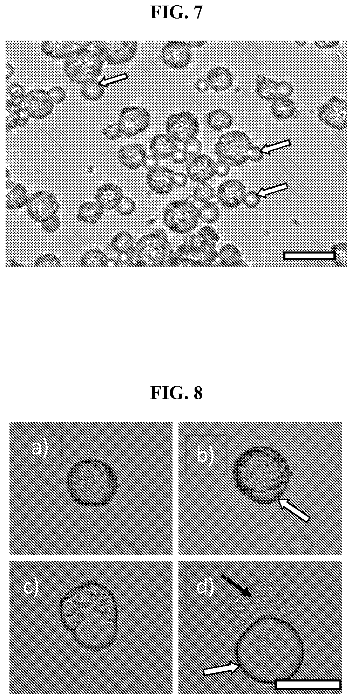

L'émulsion $E_{0,67}$ de la figure 3b) a ensuite été observée par microscopie optique à l'aide du microscope à platine chauffante Mettler afin d'étudier la rupture de l'enveloppe de silice sous l'effet de l'élévation de la température à une température supérieure à 37°C, c'est-à-dire à une température supérieure à la température de fusion de l'eicosane. L'image correspondante est donnée par la figure 7 annexée sur laquelle les flèches blanches montrent les gouttelettes d'eicosane libérées dans l'eau suite à la rupture de l'enveloppe ; la barre d'échelle correspond à 60 $\mu$m.

L'émulsion $P_{42-44}$ a également été observée par microscopie optique tout en étant soumise à une élévation de la température à une vitesse de 5°C par min. jusqu'à une température de 60°C. Les images correspondantes sont données par la figure 8 annexée sur laquelle l'image 8a) correspondant à la photographie prise à 35°C (température inférieure à la température de fusion de la paraffine 42-44, l'image 8b) à la photographie prise à 50°C (température supérieure à la température de fusion de la paraffine 42-44), l'image 8c) à la photographie prise à 55°C et la photo 8d) à la photographie prise à 60°C. Sur cette figure, la barre d'échelle correspond à 20 $\mu$m, les flèches blanches montrent la goutte d'huile sortant de l'enveloppe de silice, la flèche noire montre l'enveloppe de silice vide après libération de l'huile. Cette figure montre que lorsque la température devient supérieure à la température de fusion de l'huile cristallisable enfermée à l'intérieur de l'enveloppe de silice, on observe la rupture de l'enveloppe causée par l'expansion thermique de l'huile permettant ainsi sa libération.

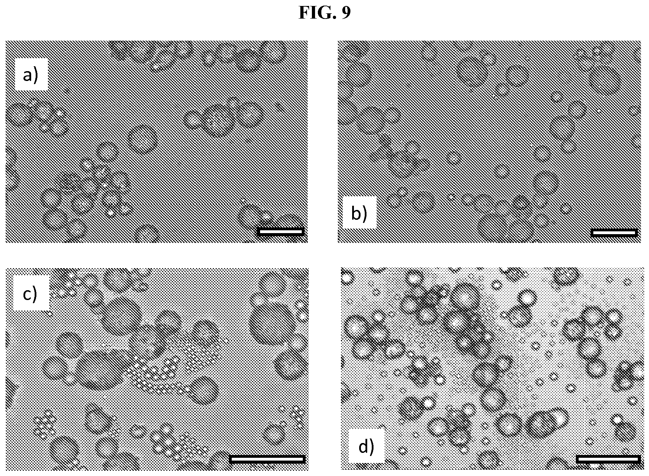

Enfin, les effets de la présence de différents tensioactifs (en une quantité suffisante pour atteindre 60 fois la concentration micellaire critique) sur la libération de l'huile ont également été étudiés pour le matériau obtenu par minéralisation de l'émulsion $E_{0,67}$. Les résultats correspondants sont donnés sur la figure 9 annexée qui représentent des images de microscopie optique de la libération des gouttelettes d'eicosane après chauffage de 33 à 53°C selon une vitesse de montée en température de 5°C/min, lors de la libération d'eicosane sans ajout de tensioactif (figure 9a), en présence d'un tensioactif anionique : le SDS (figure 9b), d'un tensioactif cationique : le CTAB (figure 9c) ou d'un tensioactif non ionique : l'Ifralan® D205 (figure 9d). Sur cette figure, la barre d'échelle correspond à 60 $\mu$m.

La présence d'un tensioactif favorise d'autant plus la libération de l'huile sous forme de gouttes d'autant plus petites qu'il abaisse la tension interfaciale entre l'eau et l'huile. Il est connu que la tension interfaciale entre une huile liquide comme l'octane et l'eau est égale à environ 0,75 mN/m en présence d'Ifralan D205 à une concentration supérieure à sa CMC, à environ 3,81 mN/m en présence de CTAB à une concentration supérieure à sa CMC, et à environ 10 mN/m en présence de SDS à une concentration supérieure à sa CMC.

L'addition d'une agitation ou d'un flux hydrodynamique favorise le détachement de l'huile de l'enveloppe de silice.

Il est ainsi possible de faire varier la taille des gouttelettes d'huile et le mode de libération en fonction de la présence ou non de tensioactif dans le milieu entourant le matériau conforme à l'invention lors de la rupture de l'enveloppe de silice.

**Exemple 2 : Préparation d'un matériau particulaire constitué d'un coeur de Suppocire® DM et d'une enveloppe de silice**

Dans cet exemple on illustre la préparation, la caractérisation et l'étude d'un matériau conforme à l'invention, constitué d'une enveloppe de silice renfermant une huile biocompatible constituée d'un mélange de triglycérides et vendue sous la dénomination Suppocire ® DM par la société Gattefossé.

Le protocole utilisé pour la préparation des matériaux préparés ci-dessus à l'exemple 1 a été utilisé dans cet exemple, en utilisant les composants suivants :

- Suppocire® DM : 12,4 g

- Phase aqueuse : 87,00 g

- Particules de silice fonctionnalisées telles que préparées à l'exemple 1 : 0,046 g

- Rapport massique quantité de particules/quantité d'huile : 0,0037

- CTAB : 0,008 g

- TEOS : 1 M/m$^2$.

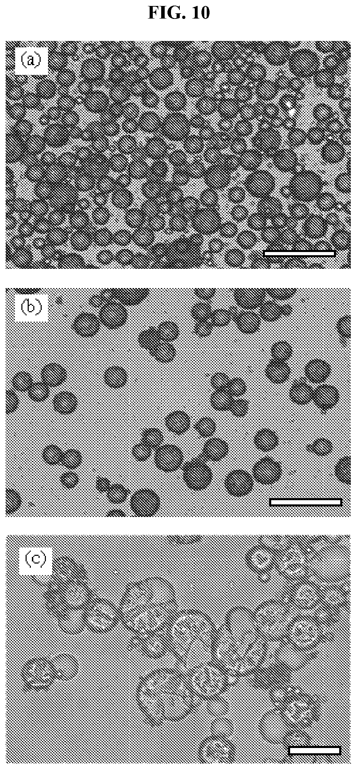

La figure 10 annexée présente des images de microscopie optique de a) l'émulsion de Suppocire® DM b) du matériau obtenu après minéralisation et c) des capsules lors de l'élévation de la température à une température de 55°C. Sur ces photographies, la barre d'échelle représente 60 $\mu$m.

**Revendications**

1. Matériau sous forme d'une poudre de particules solides sphériques ou substantiellement sphériques constituées d'une enveloppe continue comprenant au moins un oxyde de silicium, ladite enveloppe forme une capsule encapsulant au moins une phase grasse, ledit matériau étant **caractérisé en ce que** ladite phase grasse est solide à la température de stockage dudit matériau et comprend majoritairement une huile cristallisable ayant une température de fusion ($T_F$) inférieure à 100°C et au moins une substance d'intérêt, l'enveloppe d'oxyde de silicium a une épaisseur de 0,1 à 2 $\mu$m et **en ce que** le diamètre desdites particules varie de 1 $\mu$m à 1 cm.

2. Matériau selon la revendication 1, **caractérisé en ce que** l'huile cristallisable est choisie parmi les matières grasses et les mélanges de matières grasses, d'origine naturelle ou synthétique, dont le point de fusion est supérieur à 15°C.

3. Matériau selon la revendication 2, **caractérisé en ce que** ledit point de fusion varie de 20 à 50°C.

4. Matériau selon l'une quelconque des revendications précédentes, **caractérisé en ce que** l'huile cristallisable représente 50 à 99,9 % en poids de la phase grasse.

5. Matériau selon l'une quelconque des revendications précédentes, **caractérisé en ce que** l'huile cristallisable est choisie parmi les paraffines, les triglycérides ; les acides gras ; les colophanes ; les cires ; les huiles végétales hydrogénées ainsi que leurs mélanges ; les bitumes synthétiques ; et leurs mélanges.

6. Matériau selon la revendication 1, **caractérisé en ce que** diamètre des particules varie de 5 $\mu$m à 500 $\mu$m.

7. Matériau selon l'une quelconque des revendications précédentes, **caractérisé en ce que** l'enveloppe comprend en outre un ou plusieurs oxydes métalliques de formule MeO$_2$ dans laquelle Me est un métal choisi parmi Zr, Ti, Th, Nb, Ta, V, W et Al.

8. Matériau selon l'une quelconque des revendications précédentes, **caractérisé en ce que** la substance d'intérêt est choisie parmi les médicaments, les principes actifs utilisables en cosmétique, les réactifs chimiques, les colorants, les pigments et les encres.

9. Matériau selon la revendication 8, **caractérisé en ce que** la substance d'intérêt est choisie parmi les médicaments choisis parmi les bactéricides, les anti inflammatoires, les analgésiques, les laxatifs locaux, les hormones et les protéines : les principes actifs cosmétiques choisis parmi les vitamines, les filtres solaires, les antioxydants, les parfums, les agents absorbeurs d'odeur, les agents déodorants, les agents anti transpirants, les colorants, les pigments, les émollients et les agents hydratants ; les réactifs chimiques choisis parmi les réactifs colorés, les indicateurs colorés, les catalyseurs, les amorceurs de polymérisation, les monomères et les complexants.

10. Matériau selon l'une quelconque des revendications précédentes, **caractérisé en ce que** la ou les substances d'intérêt représentent de 0,1 à 50 % en poids du poids total de la phase grasse.

11. Procédé de préparation d'un matériau tel que défini à l'une quelconque des revendications 1 à 10, **caractérisé en ce qu'**il comporte les étapes suivantes consistant :

    1) dans une première étape, à porter une phase grasse comprenant majoritairement une huile cristallisable (HC) solide ayant une température de fusion $T_F$ inférieure à 100°C à une température $T_{HC}$ telle que $T_{HC}$ est supérieure à $T_F$, pour obtenir une huile cristallisable à l'état liquide ;
    2) dans une deuxième étape, à incorporer à la phase grasse à l'état liquide au moins une substance d'intérêt ;
    3) dans une troisième étape, à mettre en contact ladite phase grasse à l'état liquide avec une phase aqueuse (PA) préalablement portée à une température $T_{PA}$ telle que $T_{PA}$ est supérieure à $T_F$, ladite phase aqueuse renfermant en outre des particules solides colloïdales ;

4) dans une quatrième étape, à soumettre le mélange liquide résultant de la troisième étape à une agitation mécanique pour obtenir une émulsion huile-dans eau formée de gouttelettes de phase grasse à l'état liquide dispersées dans la phase aqueuse continue et dans laquelle les particules solides colloïdales sont présentes à l'interface formée entre la phase aqueuse continue et les gouttelettes de phase grasse dispersées ;

5) dans une cinquième étape, à porter ladite émulsion H/E à une température $T_{H/E}$ telle que $T_{H/E}$ est inférieure à $T_F$ pour provoquer la solidification de la phase grasse et obtenir une émulsion H/E formée de globules de phase grasse à l'état solide, lesdits globules étant dispersés dans la phase aqueuse ;

6) dans une sixième étape, à former une enveloppe continue comprenant au moins un oxyde de silicium autour desdits globules par ajout, dans la phase aqueuse de l'émulsion, et sous agitation mécanique, d'au moins un précurseur d'oxyde de silicium et d'une quantité suffisante d'au moins un acide pour amener la phase aqueuse à pH inférieur ou égal à 4 pour obtenir ledit matériau ; et

7) dans une septième étape, à séparer ledit matériau de la phase aqueuse.

12. Procédé selon la revendication 11, **caractérisé en ce que** les particules solides colloïdales sont des particules minérales choisies dans le groupe des oxydes, hydroxydes et sulfates de métaux.

13. Procédé selon la revendication 12, **caractérisé en ce que** les oxyde de métaux sont choisis parmi les oxydes de silicium, de titane, de zirconium et de fer ; et leur sels.

14. Procédé selon la revendication 13, **caractérisé en ce que** les particules sont choisies parmi les nanoparticules d'oxyde de silicium.

15. Procédé selon l'une quelconque des revendications 11 à 14, **caractérisé en ce que** la quantité de particules solides colloïdales varie de 0,01 % à 10 % en poids par rapport au poids total de la phase grasse.

16. Procédé selon l'une quelconque des revendications 11 à 15, **caractérisé en ce que** les particules solides colloïdales sont fonctionnalisées par adsorption de molécules de tensioactif à leur surface.

17. Procédé selon l'une quelconque des revendications 11 à 16, **caractérisé en ce que** les précurseurs de silice sont choisis parmi les alkoxydes de silice.

18. Procédé selon la revendication 17, **caractérisé en ce que** les alcoxydes de silice sont choisis parmi le tetraméthoxyorthosilane, le tetraéthoxyorthosilane, le diméthyldiéthoxysilane, le (3-mercaptopropyl)triméthoxysilane, le (3-aminopropyl)triéthoxysilane, le N-(3-triméthoxysilylpropyl)pyrrole, le 3-(2,4-dinitrophénylamino)-propyltriéthoxysilane, le N-(2-aminoéthyl)-3-aminopropyltriméthoxysilane, le phényltriéthoxysilane, le méthyltriéthoxysilane et leurs mélanges.

19. Procédé selon l'une quelconque des revendications 11 à 18, **caractérisé en ce que** la quantité de précurseur d'oxyde de silicium varie de 0,05 à 4 $M/m^2$ de surface des globules de la phase dispersée de l'émulsion.

20. Procédé selon l'une quelconque des revendications 11 à 19, **caractérisé en ce que** le pH de la phase aqueuse lors de la sixième étape varie de 0,1 à 2,1.

21. Utilisation d'un matériau tel que défini à l'une quelconque des revendications 1 à 10, sous forme de poudre ou de dispersion dans un solvant, pour la délivrance thermostimulée d'au moins une substance d'intérêt.

22. Utilisation selon la revendication 21 **caractérisée en ce que** la délivrance thermostimulée est obtenue par rupture de l'enveloppe sous l'effet d'une élévation de la température à une température de délivrance $T_D$ telle que $T_D > T_F$.

23. Utilisation d'un matériau tel que défini à l'une quelconque des revendications 1 à 10, à titre d'ingrédient, pour la préparation de produits pharmaceutiques, cosmétiques ou alimentaires.

24. Produits pharmaceutiques, cosmétiques ou alimentaires, **caractérisés en ce qu'**ils renferment, à titre d'ingrédient, au moins un matériau tel que défini à l'une quelconque des revendications 1 à 10.

**EP 2 459 306 B1**

**Patentansprüche**

1. Material in Form eines Pulvers aus kugeligen oder substantiell kugeligen festen Partikeln, gebildet von einer kontinuierlichen Hülle, umfassend mindestens ein Siliziumoxid, wobei die Hülle eine Kapsel bildet, die mindestens eine Fettphase einkapselt, wobei das Material **dadurch gekennzeichnet ist, dass** die Fettphase bei Lagertemperatur des Materials fest ist und mehrheitlich ein kristallisierbares Öl mit einer Schmelztemperatur ($T_F$) unter 100 °C und mindestens eine interessierende Substanz umfasst, wobei die Siliziumoxidhülle eine Stärke von 0,1 bis 2 $\mu$m hat und dass der Durchmesser der Partikel von 1 $\mu$m bis 1 cm schwankt.

2. Material nach Anspruch 1, **dadurch gekennzeichnet, dass** das kristallisierbare Öl aus den Fetten und den Fettgemischen natürlichen oder synthetischen Ursprungs ausgewählt ist, deren Schmelzpunkt über 15 °C liegt.

3. Material nach Anspruch 2, **dadurch gekennzeichnet, dass** der Schmelzpunkt von 20 bis 50 °C schwankt.

4. Material nach einem der vorangehenden Ansprüche, **dadurch gekennzeichnet, dass** das kristallisierbare Öl 50 bis 99,9 Gew.-% der Fettphase darstellt.

5. Material nach einem der vorangehenden Ansprüche, **dadurch gekennzeichnet, dass** das kristallisierbare Öl aus den Paraffinen, den Triglyceriden, den Fettsäuren, den Colophanen, den Wachsen, den hydrogenierten Pflanzenölen sowie ihren Gemischen, den synthetischen Bitumen und ihren Gemischen ausgewählt ist.

6. Material nach Anspruch 1, **dadurch gekennzeichnet, dass** der Durchmesser der Partikel von 5 $\mu$m bis 500 $\mu$m schwankt.

7. Material nach einem der vorangehenden Ansprüche, **dadurch gekennzeichnet, dass** die Hülle ferner ein oder mehrere Metalloxide der Formel $MeO_2$ umfasst, wobei Me ein Metall ist, das aus Zr, Ti, Th, Nb, Ta, V, W und Al ausgewählt ist.

8. Material nach einem der vorangehenden Ansprüche, **dadurch gekennzeichnet, dass** die interessierende Substanz aus den Arzneimitteln, den in der Kosmetik verwendbaren Wirkstoffen, den chemischen Reagenzien, den Farbstoffen, den Pigmenten und den Farben ausgewählt ist.

9. Material nach Anspruch 8, **dadurch gekennzeichnet, dass** die interessierende Substanz aus den Arzneimitteln, ausgewählt aus den Bakteriziden, den Entzündungshemmern, den Analgetika, den Lokallaxativa, den Hormonen und den Proteinen, den kosmetischen Wirkstoffen, ausgewählt aus den Vitaminen, den Sonnenfiltern, den Antioxidantien, den Duftstoffen, den Geruchsvernichtern, den Deodorants, den Antitranspirantien, den Farbstoffen, den Pigmenten, den Weichmachern und den Feuchtigkeitsspendern, den chemischen Reagenzien, ausgewählt aus den Farbreagenzien, den Farbindikatoren, den Katalysatoren, den Polymerisationsstartern, den Monomeren und den Komplexbildnern, ausgewählt ist.

10. Material nach einem der vorangehenden Ansprüche, **dadurch gekennzeichnet, dass** die interessierende(n) Substanz(en) 0,1 bis 50 Gew.-% des Gesamtgewichts der Fettphase darstellen.

11. Herstellungsverfahren eines Materials nach einem der Ansprüche 1 bis 10, **dadurch gekennzeichnet, dass** es die folgenden Schritte umfasst:

    1) in einem ersten Schritt, Verbringen einer Fettphase, die mehrheitlich ein festes kristallisierbares Öl (HC) mit einer Schmelztemperatur $T_F$ unter 100 °C umfasst, auf eine Temperatur $T_{HC}$, die derart ist, dass $T_{HC}$ größer als $T_F$ ist, um ein kristallisierbares Öl in flüssigem Zustand zu erhalten,
    2) in einem zweiten Schritt, Einarbeiten in die Fettphase in flüssigem Zustand mindestens einer interessierenden Substanz,
    3) in einem dritten Schritt, Inkontaktversetzen der Fettphase in flüssigem Zustand mit einer Wasserphase (PA), die zuvor auf eine Temperatur $T_{PA}$ gebracht wurde, die derart ist, dass $T_{PA}$ größer als $T_F$ ist, wobei die Wasserphase ferner kolloidale feste Partikel einschließt,
    4) in einem vierten Schritt, mechanisches Rühren des flüssigen Gemischs im Ergebnis des dritten Schritts, um eine Öl-in-Wasser-Emulsion zu erhalten, die von Tröpfchen der Fettphase in flüssigem Zustand gebildet ist, die in der kontinuierlichen Wasserphase dispergiert sind, und in welcher die kolloidalen festen Partikel an der Schnittstelle vorhanden sind, die zwischen der kontinuierlichen Wasserphase und den dispergierten Tröpfchen der Fettphase gebildet ist,

5) in einem fünften Schritt, Verbringen der Ö/W-Emulsion auf eine Temperatur $T_{H/E}$, die derart ist, dass $T_{H/E}$ kleiner als $T_F$ ist, um die Verfestigung der Fettphase auszulösen und eine Ö/W-Emulsion zu erhalten, die von Fettphasekügelchen in festem Zustand gebildet ist, wobei die Kügelchen in der Wasserphase dispergiert sind,

6) in einem sechsten Schritt, Bilden einer kontinuierlichen Hülle, die mindestens ein Siliziumoxid um die Kügelchen umfasst, durch Hinzufügen, in die Wasserphase der Emulsion und unter mechanischem Rühren, von mindestens einem Siliziumoxidvorläufer und einer ausreichenden Menge mindestens einer Säure, um die Wasserphase auf einen pH unter oder von gleich 4 zu führen, um das Material zu erhalten, und

7) in einem siebenten Schritt, Separieren des Materials von der Wasserphase.

**12.** Verfahren nach Anspruch 11, **dadurch gekennzeichnet, dass** die kolloidalen festen Partikel mineralische Partikel sind, die aus der Gruppe der Oxide, Hydroxide und Sulfate von Metallen ausgewählt sind.

**13.** Verfahren nach Anspruch 12, **dadurch gekennzeichnet, dass** die Metalloxide aus den Oxiden von Silizium, Titan, Zirkonium und Eisen und ihren Salzen ausgewählt sind.

**14.** Verfahren nach Anspruch 13, **dadurch gekennzeichnet, dass** die Partikel aus den Siliziumoxid-Nanopartikeln ausgewählt sind.

**15.** Verfahren nach einem der Ansprüche 11 bis 14, **dadurch gekennzeichnet, dass** die Menge der kolloidalen festen Partikel von 0,01 % bis 10 Gew.-% in Bezug zum Gesamtgewicht der Fettphase schwankt.

**16.** Verfahren nach einem der Ansprüche 11 bis 15, **dadurch gekennzeichnet, dass** die kolloidalen festen Partikel durch Adsorption von Tensidmolekülen auf ihrer Oberfläche funktionalisiert sind.

**17.** Verfahren nach einem der Ansprüche 11 bis 16, **dadurch gekennzeichnet, dass** die Siliziumvorläufer aus den Siliziumoxidalkoxiden ausgewählt sind.

**18.** Verfahren nach Anspruch 17, **dadurch gekennzeichnet, dass** die Siliziumoxidalkoxide aus dem Tetramethoxyorthosilan, dem Tetraethoxyorthosilan, dem Dimethyldiethoxysilan, dem (3-Mercaptopropyl)trimethoxysilan, dem (3-Aminpropyl)triethoxysilan, dem N-(3-trimethoxysilylpropyl)pyrrol, dem 3-(2,4-dinitrophenylamin)-propyltriethoxysilan, dem N-(2-aminethyl)-3-aminpropyltrimethoxysilan, dem Phenyltriethoxysilan, dem Methyltriethoxysilan und ihren Gemischen ausgewählt sind.

**19.** Verfahren nach einem der Ansprüche 11 bis 18, **dadurch gekennzeichnet, dass** die Menge Siliziumoxidvorläufer von 0,05 bis 4 $M/m^2$ Oberfläche der Kügelchen der dispergierten Phase der Emulsion schwankt.

**20.** Verfahren nach einem der Ansprüche 11 bis 19, **dadurch gekennzeichnet, dass** der pH der Wasserphase beim sechsten Schritt von 0,1 bis 2,1 schwankt.

**21.** Verwendung eines Materials nach einem der Ansprüche 1 bis 10 in Form von Pulver oder von Dispersion in einem Lösungsmittel für die thermisch stimulierte Ausgabe mindestens einer interessierenden Substanz.

**22.** Verwendung nach Anspruch 21, **dadurch gekennzeichnet, dass** die thermisch stimulierte Ausgabe durch Riss der Hülle unter der Wirkung einer Temperaturerhöhung auf eine Ausgabetemperatur $T_D$ erfolgt, die derart ist, dass $T_D > T_F$ ist.

**23.** Verwendung eines Materials nach einem der Ansprüche 1 bis 10 als Inhaltsstoff für die Zubereitung von pharmazeutischen, kosmetischen oder Lebensmittelprodukten.

**24.** Pharmazeutische, kosmetische oder Lebensmittelprodukte, **dadurch gekennzeichnet, dass** sie als Inhaltsstoff mindestens ein Material nach einem der Ansprüche 1 bis 10 einschließen.

**Claims**

**1.** Material in the form of a powder of solid spherical or substantially spherical particles composed of a continuous shell comprising at least one silicon oxide, said shell forms a capsule encapsulating at least one fat phase, said material being **characterized in that** said fat phase is solid at the storage temperature of said material and mostly comprises a crystallisable oil having a melting point ($T_M$) lower than 100°C and at least one substance of interest, the silicon oxide shell has a thickness of 0.1 to 2 $\mu$m, and **in that** the diameter of said particles varies from 1 $\mu$m to 1 cm.

2. The material according to claim 1, **characterized in that** the crystallisable oil is selected from among fats and mixtures of fats of natural or synthetic origin, having a melting point higher than 15°C.

3. The material according to claim 2, **characterized in that** said melting point varies from 20 to 50°C.

4. The material according to any of the preceding claims, **characterized in that** the crystallisable oil represents 50 to 99.9 % by weight of the fat phase.

5. The material according to any of the preceding claims, **characterized in that** the crystallisable oil is selected from among paraffins, triglycerides; fatty acids; colophons; waxes; hydrogenated vegetable oils and mixtures thereof; synthetic bitumens; and mixtures thereof.

6. The material according to claim 1, **characterized in that** the diameter of the particles varies from 5 $\mu$m to 500 $\mu$m.

7. The material according to any of the preceding claims, **characterized in that** the shell also comprises one or more metal oxides of formula $MeO_2$ where Me is a metal selected from among Zr, Ti, Th, Nb, Ta, V, W and Al.

8. The material according to any of the preceding claims, **characterized in that** the substance of interest is selected from among medicinal products, active ingredients useable in cosmetics, chemical reagents, dyes, pigments and inks.

9. The material according to claim 8, **characterized in that** the substance of interest is selected from among medicinal products selected from among bactericides, anti-inflammatories, analgesics, local laxatives, hormones and proteins; cosmetic active ingredients selected from among vitamins, sun filters, antioxidants, fragrances, odour-absorbing agents, deodorants, antiperspirants, colouring agents, pigments, emollients and hydrating agents; chemical reagents selected from among staining reagents, colour indicators, catalysts, polymerisation initiators, monomers and complexing agents.

10. The material according to any of the preceding claims, **characterized in that** the substance(s) of interest represent 0.1 to 50 % by weight of the total weight of the fat phase.

11. Method for preparing a material such as defined in any of claims 1 to 10, **characterized in that** it comprises the following steps of:

1) at a first step, bringing a fat phase, mostly comprising a solid crystallisable oil (CO) having a melting point $T_M$ lower than 100 °C, to a temperature $T_{CO}$ such that $T_{CO}$ is higher than $T_M$ to obtain a crystallisable oil in the liquid state;
2) at a second step, incorporating at least one substance of interest in the fat phase in the liquid state.
3) at a third step, contacting said fat phase in the liquid state with an aqueous phase (AP) previously brought to a temperature $T_{AP}$ such that $T_{AP}$ is higher than $T_M$, said aqueous phase also containing solid colloidal particles;
4) at a fourth step, subjecting the liquid mixture resulting from the third step to mechanical stirring to obtain an oil-in-water emulsion formed of droplets of fat phase in the liquid state dispersed in the continuous aqueous phase, and in which the solid colloidal particles are present on the interface formed between the continuous aqueous phase and the dispersed droplets of fat phase;
5) at a fifth step, bringing said O/W emulsion to a temperature $T_{O/W}$ such that $T_{O/W}$ is lower than $T_M$ to cause solidification of the fat phase and obtain an O/W emulsion formed of globules of fat phase in the solid state, said globules being dispersed in the aqueous phase;
6) at a sixth step, forming a continuous shell comprising at least one silicon oxide around said globules by adding to the aqueous phase of the emulsion, under mechanical stirring, at least one silicon oxide precursor and a sufficient quantity of at least one acid to bring the aqueous phase to a pH of 4 or lower to obtain said material, and
7) at a seventh step, separating said material from the aqueous phase.

12. The method according to claim 11, **characterized in that** the solid colloidal particles are mineral particles selected from the group of metal oxides, hydroxides and sulfates.

13. The method according to claim 12, **characterized in that** the metal oxides are selected from among oxides of silicon, titanium, zirconium and iron; and salts thereof.

14. The method according to claim 13, **characterized in that** the particles are selected from among nanoparticles of silicon oxide.

15. The method according to any of claims 11 to 14, **characterized in that** the quantity of solid colloidal particles varies from 0.01 % to 10 % by weight relative to the total weight of the fat phase.

16. The method according to any of claims 11 to 15, **characterized in that** the solid colloidal particles are functionalised by adsorption of surfactant molecules on their surface.

17. The method according to any of claims 11 to 16, **characterized in that** the silicon precursors are selected from among silicon alkoxides.

18. The method according to claim 17, **characterized in that** the silicon alkoxides are selected from among tetramethoxyorthosilane, tetraethoxyorthosilane, dimethyl diethoxysilane, (3-mercaptopropyl)trimethoxysilane, (3-aminopropyl)triethoxysilane, N-(3-trimethoxysilylpropyl)pyrrole, 3-(2,4-dinitrophenylamino)-propyltriethoxysilane, N-(2-aminoethyl)-3-aminopropyltrimethoxysilane, phenyltriethoxysilane, methyltriethoxysilane and mixtures thereof.

19. The method according to any of claims 11 to 18, **characterized in that** the quantity of silicon oxide precursor varies from 0.05 to $4M/m^2$ surface area of the globules of the phase dispersed in the emulsion.

20. The method according to any of claims 11 to 19, **characterized in that** the pH of the aqueous phase at the sixth step varies from 0.1 to 2.1.

21. Use of a material such as defined in any of claims 1 to 10, in the form of a powder or dispersion in a solvent, for thermostimulated delivery of at least one substance of interest.

22. The use according to claim 21, **characterized in that** thermostimulated delivery is obtained by rupture of the shell under the effect of a rise in temperature to a delivery temperature $T_D$ such that $T_D > T_M$.

23. The use of a material such as defined in any of claims 1 to 10 as ingredient to prepare pharmaceutical, cosmetic or food products.

24. Pharmaceutical, cosmetic or food products, **characterized in that** as ingredient they contain at least one material such as defined in any of claims 1 to 10.

Fig. 1

**Fig. 2**

**FIG. 3**

## FIG. 4

**FIG. 5**

**FIG. 6**

FIG. 7

FIG. 8

**FIG. 9**

**FIG. 10**

**EP 2 459 306 B1**

**RÉFÉRENCES CITÉES DANS LA DESCRIPTION**

**Documents brevets cités dans la description**

• WO 2008072239 A2